# EUROPEAN PATENT APPLICATION

(11) **EP 3 273 238 A1**
(43) Date of publication of application: **24.01.2018**
(21) Application number: 16180866.2
(22) Date of filing: 22.07.2016
(51) Int. Cl.: G01N 33/50, G01N 33/564

(54) **IMMUNODIAGNOSTIC DETECTION OF ANTI-NEUTROPHIL ANTIBODIES**

(71) Applicant: Universitätsklinikum Hamburg-Eppendorf, 20246 Hamburg (DE)
(72) Inventor: Fuchs, Tobias, 20249 Hamburg (DE)
(74) Representative: Stüven, Ralf

(57) **Abstract**

The invention relates to immunodiagnostic detection of anti-neutrophil antibodies. Object of the current invention is to improve the diagnosis of ANCA-associated diseases, in particular ANCA-associated vasculitides. For solving the problem, the invention provides in one aspect a method for the immunodiagnostic detection of anti-neutrophil antibodies, the method comprising:
a1) Contacting an aliquot of a sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
a2) Immunolabeling of anti-neutrophil antibodies bound to the stimulated netotic but not netting polymorphonuclear neutrophils.

In further aspects the invention provides a method for preparing an object slide for the immunodiagnostic detection of anti-neutrophil antibodies, an object slide for the immunodiagnostic detection of anti-neutrophil antibodies, a kit for the immunodiagnostic detection of anti-neutrophil antibodies, and the use of stimulated neutrophils for the immunodiagnostic detection of anti-neutrophil antibodies.

## Description

The invention relates to a method for the immunodiagnostic detection of anti-neutrophil antibodies, a method for preparing an object slide for the immunodiagnostic detection of anti-neutrophil antibodies, an object slide for the immunodiagnostic detection of anti-neutrophil antibodies, a kit for the immunodiagnostic detection of anti-neutrophil antibodies, and the use of stimulated neutrophils for the immunodiagnostic detection of anti-neutrophil antibodies.

Antibodies against neutrophil granulocytes play an important role in the diagnosis of autoimmune-related diseases, such as inflammatory vascular disease (primary vasculitis) and primary sclerosing cholangitis. These autoantibodies mainly target components of the cytoplasmic neutrophil secretory granules, and are therefore referred to as anti-neutrophil cytoplasmic antibodies, ANCAs (F. J. Vanderwoude et al., 1985, Autoantibodies against Neutrophils and Monocytes - Tool for Diagnosis and Marker of Disease-Activity in Wegeners Granulomatosis, Lancet 1, 425-429; R. J. Falk, J. C. Jennette, 1988, Anti-neutrophil cytoplasmic autoantibodies with specificity for myeloperoxidase in patients with systemic vasculitis and idiopathic necrotizing and crescentic glomerulonephritis, N Engl J Med 318, 1651-1657; J. L. Niles, R. T. McCluskey, M. F. Ahmad, M. A. Arnaout, 1989, Wegener's granulomatosis autoantigen is a novel neutrophil serine proteinase, Blood 74, 1888-1893).

Although linked to several inflammatory conditions, ANCAs are primarily used for the diagnosis of primary vasculitis, namely granulomatosis with polyangiitis (GPA) and microscopic polyangiitis (MPA) (J. C. Jennette et al., 1994, Nomenclature of Systemic Vasculitides - Proposal of an International Consensus Conference, Arthritis and Rheumatism 37, 187-192). Since ANCAs are an important factor both in pathogenesis and diagnosis of primary vasculitis, the term ANCA-associated vasculitides (AAV) is often used in this context.

Methods for the immunodiagnostic detection of anti-neutrophil cytoplasmic antibodies are generally known. In a frequently used method, indirect immunofluorescence (IIF), ethanol-fixed naive, i.e. unstimulated, neutrophils are incubated with potentially ANCA-containing patient serum and bound ANCA are detected by means of fluorescently labeled secondary antibodies directed against them (see, for example, Savige et al. 1999, International Consensus Statement on Testing and Reporting of antineutrophil cytoplasmic Antibodies (ANCA), J Clin Pathol 111 Am: 507-13; Savige J, et al 2003, Addendum to the International Consensus statement on testing and reporting of antineutrophil cytoplasmic antibodies Quality control guidelines, comments, and recommendations for testing in other autoimmune diseases, American Journal of Clinical Pathology, 120, 312-318, doi: 10.1309/WAEPADW0K4LPUHFN; Csernok E and Moosig F, 2014, Current and emerging techniques for detection ANCA in vasculitis, Nature Reviews Rheumatology 10, 494-501, doi: 10.1038/nrrheum.2014.78; Schulte-Pelkum J, et al 2014, Novel clinical and diagnostic aspects of antineutrophil cytoplasmic antibodies, J Immunol Res. 2014, doi: 10.1155/2014/185416). With indirect immunofluorescence (IFF) of ethanol-fixed naive neutrophils two main groups of intracellular staining patterns are found, a cytoplasmic dotted pattern (c-ANCA) and a perinuclear pattern with accentuation of the nuclear segments (p-ANCA). The c-ANCA pattern is based on the binding of autoantibodies against proteinase 3 (PR3), and is primarily associated with GPA, while the p-ANCA pattern is mainly due to the reaction of autoantibodies against myeloperoxidase (MPO) and mainly associated with MPA. In naive cells, both PR3 and MPO are stored in azurophilic granules. For still unknown reasons, ethanol used for fixing the neutrophils causes the redistribution of myeloperoxidase (MPO) to the periphery of the nucleus (p-ANCA), whereas proteinase 3 (PR3) remains within the cytoplasm (c-ANCA) (R. J. Falk, J. C. Jennette, 1988, Anti-neutrophil cytoplasmic autoantibodies with specificity for myeloperoxidase in patients with systemic vasculitis and idiopathic necrotizing and crescentic glomerulonephritis, N Engl J Med 318, 1651-1657). The p-ANCA staining pattern seen with ethanol-fixed neutrophils is thus a fixation artifact, which does not occur with cells fixed with e.g. formalin.

While the methods used for diagnosing AAV are usually carried out with naïve neutrophils, Dwivedi et al. 2012 describe experiments in which the sera of patients with systemic lupus erythematosus (SLE), rheumatoid arthritis (RA), Felty's syndrome (FS) and AAVs have been investigated for binding to activated neutrophils, deiminated histones and Neutrophil Extracellular Traps (NETs) (Dwivedi N. et al., 2012, Felty's syndrome autoantibodies bind to histones and deiminated neutrophil extracellular traps chromatin, Arthritis Rheum. 64: 982-92, doi: 10.1002/art.33432). Neutrophil Extracellular Traps (NETs) are lattices of extracellular DNA filaments containing histones and enzymes from neutrophil cytoplasmic granules (V. Brinkmann et al., 2004, Neutrophil extracellular traps kill bacteria, Science 303, 1532-1535). NETs are released by activated neutrophils and display diverse functions in host defence against infection, sterile inflammatory diseases, and autoimmunity (O. E. Sorensen, N. Borregaard, 2016, Neutrophil extracellular traps - the dark side of neutrophils, The Journal of clinical investigation 126, 1612-1620). Neutrophils form NETs by a multi-step cell death program (NETosis), which involves the breakdown of internal membranes, karyolysis, and the rupture of the plasma membrane (T. A. Fuchs et al., 2007, Novel cell death program leads to neutrophil extracellular traps, Journal of Cell Biology 176, 231-241; V. Papayannopoulos, K. D. Metzler, A. Hakkim, A. Zychlinsky, 2010, Neutrophil elastase and myeloperoxidase regulate the formation of neutrophil extracellular traps, Journal of Cell Biology 191, 677-691).

IFF is usually done manually and is therefore time-consuming, subjective and requires expertise. The identification of ANCA subtypes is difficult and prone to error, thus limiting the clinical benefit of ANCA diagnosis. Further, with the existing procedures, ANCAs are not detected in all patients with an ANCA-associated disease. In about 15% of patients with ANCA-associated vasculitis no ANCA are detected, and the term ANCA-negative vasculitis is used in this context (Chen M, Kallenberg CG, Zhao MH, 2009, ANCA-negative pauci-immune crescentic glomerulonephritis, Nat Rev Nephrol. 2009, 5(6): 313-318, doi: 10.1038/nrneph.2009.67; Kallenberg CGM 2014, Key advances in the clinical approach to ANCA-associated vasculitis, Nature Reviews Rheumatology 10, 484-493, doi: 10.1038/nrrheum.2014.104). ANCA diagnostic findings therefore have only a limited informative value.

Further, with ethanol-fixed neutrophils p-ANCAs can easily be confused with antinuclear antibodies (ANAs), because of the very similar staining pattern in IFF. For the discrimination between p-ANCAs and ANAs further diagnostic tests are therefore necessary, e.g. ELISA or IFF analysis of formalin-fixed naive neutrophils (Lee SS, Lawton JW, Chak W., 1991, Distinction between antinuclear antibody and P-ANCA, J Clin Pathol. 44(11): 962-963).

Object of the current invention is to improve the diagnosis of ANCA-associated diseases, in particular ANCA-associated vasculitides.

In a first aspect the invention provides a method for the immunodiagnostic detection of anti-neutrophil antibodies, the method comprising:
a1) Contacting an aliquot of a sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
a2) Immunolabeling of anti-neutrophil antibodies bound to the stimulated netotic but not netting polymorphonuclear neutrophils.

It has surprisingly been found that, by immunolabeling stimulated netotic but not netting polymorphonuclear neutrophils instead of naïve, i.e. unstimulated, polymorphonuclear neutrophils, wherein the netotic but not netting polymorphonuclear neutrophils have been generated by treating stimulated, but not netting, polymorphonuclear neutrophils with a suitable NET release inhibiting compound, for example diisopropylfluorophosphate, DFP, under conditions where the formation of NETs by the stimulated polymorphonuclear neutrophils is suppressed, sensitivity and/or specificity of ANCA testing can significantly be improved. Further, the invention enables a considerably improved discrimination between c-ANCAs and p-ANCAs. The invention is particularly useful for (differential) diagnosis of ANCA-associated vasculitides, AAVs, e.g. for the diagnosis of granulomatosis with polyangiitis or microscopic polyangiitis, but is not limited thereto. It is a further advantage of the inventive method that it can at least partly be carried out automatically, thus further improving the reliability of ANCA testing.

Without wishing to be bound by theory, it is believed that during the stimulation of PMNs altered cellular components and neo-antigens are formed. The known methods do not detect antibodies to these neo-antigens and may therefore lead to false negative ANCA findings. In contrast, the invention also provides for the detection of antibodies directed against altered cellular components in stimulated neutrophils. Further, it is believed that a suitable NET release inhibiting compound like the serine proteinase inhibitor diisopropylfluorophosphate, DFP, blocks the disintegration of the nucleus and the generation of NETs during NETosis without preventing the nuclear translocation of MPO. It is believed that DFP blocks neutrophil elastase (NE), which is required for this process and mediates the unfolding of chromatin by cleaving core histones and the linker histone H1 (V. Papayannopoulos, K. D. Metzler, A. Hakkim, A. Zychlinsky, 2010, Neutrophil elastase and myeloperoxidase regulate the formation of neutrophil extracellular traps, Journal of Cell Biology 191, 677-691). Immunolabeling of stimulated PMNs treated with a NET release inhibiting compound, e.g. DFP, during NETosis, such that the otherwise occurring release of NETs is suppressed, results in clearly distinguishable c-ANCA and p-ANCA staining patterns, which can easily be evaluated by humans or automatically.

In the method according to the invention isolated unstimulated PMNs may, for example, initially be treated with the NET release inhibiting compound, e.g. DFP, and stimulated in a suitable manner, such that the PMNs enter NETosis without forming NETs, and the resulting netotic, but not netting PMNs are subsequently preferably fixed on a suitable carrier, e.g. a glass slide in a known manner, for example, with ethanol, methanol or formalin. Following steps may include steps known in the art, for example, incubating the PMNs immobilized and fixed on a glass slide with a body fluid sample, such as a blood plasma or blood serum sample, subsequently incubating (staining) the sample with fluorescently labeled anti-human IgG antibodies and appropriate washing steps.

The term "anti-neutrophil antibody" relates to antibodies, particularly autoantibodies, directed against antigens of leucocytes, in particular neutrophilic granulocytes (neutrophils) and monocytes, regardless of where these antigens are localized on or in the cells. The term also includes antibodies directed against components, which are released from the cells to the outside, e.g. against components of Neutrophil Extracellular Traps (NETs).

The term "anti-neutrophil cytoplasmic antibody", abbreviated ANCA, generally relates to antibodies directed against antigens in the cytoplasm of leukocytes, particularly neutrophils and monocytes. ANCA occur mainly in the context of various autoimmune diseases such as vasculitides. In individuals with a corresponding disease, anti-neutrophil cytoplasmic autoantibodies can be detected, i.e. antibodies formed by the body that are directed against the body's own leukocytes. A distinction between different ANCA forms is based on the fluorescence pattern observed in indirect immunofluorescence tests (indirect IFT, IIF) used for diagnosis and performed on ethanol-fixed neutrophils: If a perinuclear staining is observed the term p-ANCA is used, the term c-ANCA relates to a cytoplasmic staining, and the term a-ANCA is used for an atypical staining pattern. In this context it is noteworthy that these ANCA patterns are an artifact of the ethanol fixation regularly used. Ethanol permeabilizes the granular membrane, leading to a rearrangement of MPO to the periphery of the nucleus, whereas PR3 remains in the cytoplasmic granules (Charles, L.A., 1989, Reactivity of anti-neutrophil cytoplasmic autoantibodies with HL-60 cells, Clinical Immunology and Immunopathology 53, 243-253). The biological mechanism and the physiological significance of this artifact are unknown. The term "anti-neutrophil cytoplasmic antibodies" thus relates in particular to human autoantibodies that are directed against the body's own human neutrophils. In the context of the present invention, the terms "anti-stimulated neutrophil antibody", ASNA, or "anti-activated neutrophil antibody", AANA, may also be used in conjunction with autoantibodies directed against stimulated neutrophils or against antigens on or in stimulated neutrophilic granulocytes, or are directed against antigens which are included in cell components, which are released from stimulated neutrophilic granulocytes to the outside, for example in NETs. The term "ANETA" may be used to denote autoantibodies directed against NET components.

The term "antinuclear antibody", ANA, relates to autoantibodies binding to components of the cell nucleus. ANAs therefore show a nuclear staining pattern similar to the staining pattern observed for p-ANCAs in naïve ethanol-fixed neutrophils.

"Neutrophil granulocytes", shortly "neutrophils", or "polymorphonuclear leukocytes", PMNs, are specialized immune cells occurring in vertebrates, which belong to the non-specific innate immune system and are involved in the immune defense against microorganisms. In humans, they form the majority (50-65%) of white blood cells (leukocytes) in the peripheral blood. In the unstimulated state the cells are spherical and have a diameter of about 10-15 microns. Mature cells are "polymorphonuclear", and are characterized by three to five nuclear interconnected segments or lobes. In the cytoplasm of neutrophilic granulocytes different types of granules are present, containing different antimicrobial agents, for example, enzymes.

The terms "stimulated neutrophilic granulocytes", "stimulated neutrophils" or "stimulated PMNs" relate to neutrophilic granulocytes, which, upon an appropriate stimulus, have left their originally unstimulated (naïve) state. In particular, the term is understood to mean stimulated neutrophilic granulocytes isolated from a human or animal body, in particular a human or animal body inflicted with an autoimmune and/or inflammatory disease, or in vitro stimulated neutrophilic granulocytes. Preferably the term relates to stimulated human neutrophils. Stimulated neutrophils regularly show changes in shape, behavior and physiological state. They regularly have a non-spherical, but amorphous, amoeba-like shape and show chemotactic behavior, adhesion, degranulation, phagocytosis, production of reactive oxygen species (ROS), changes in the nuclear structure, etc. This entails physiological changes, for example in terms of the composition of the surface receptors. A comparatively weak, reversible stimulation is sometimes referred to in the art as "priming", while the term "activation" is used in relation to an irreversible stimulation, often encompassing the formation of NETs. Therefore, the process induced by (irreversible) stimulation and leading to a programmed cell death (apoptosis) of neutrophils is also called "NETosis". The term "stimulation" as used herein includes all of these meanings. In particular, however, the term here means the setting of an external stimulus, which would, without any further intervention, for example, without prior killing and/or fixation of the cells, lead to adhesion, degranulation, phagocytosis and NET formation of neutrophilic granulocytes. Alternatively or additionally, the term here includes setting a cellular process in motion, during which the cellular composition of neutrophils is changed so that it comes to a mixing of constituents, e.g. molecules, from different cellular compartments (for example, granules, nucleus, cytoplasm, mitochondria, endoplasmic reticulum). Stimulated neutrophils and methods for their in-vitro stimulation are generally known to a person skilled in the art. An example of a suitable stimulating agent is phorbol 12-myristate 13-acetate, PMA (see e.g. David A. et al. 2003, Interaction of proteinase 3 with CD11b/CD18 (β2 integrin) on the cell membrane of human neutrophils, Journal of Leukocyte Biology 74, 551-557). Other known stimulating agents are calcium ionophores (s. e.g., Godfrey et al., 1987, Stimulus-Specific Induction of phospholipid and Arachidonic Acid Metabolism in Human Neutrophils, The Journal of Cell Biology 104, 925-932). Neutrophils isolated from blood are a very heterogeneous population of cells. A subset of neutrophils also activates, for example, when kept over a long time (> 3h) unpurified in contact with foreign material, for example, are kept in cell culture dishes (self-activation or autoactivation), or are, after purification, exposed over more than 15 minutes to foreign material, such as glass or plastic surfaces. Without wishing to be bound by theory, it is believed that the stimulation of PMNs leads to the formation of new structures, which can act as antigens (neoantigens). In addition to the type of stimulation, its duration may also be relevant. It has, for example, been observed that calcium ionophores generated new antigens after 30 minutes, which - possibly due to proteolytic degradation - disappeared with prolonged stimulation. The skilled person can easily determine suitable stimulation conditions and durations by routine experimentation. A preferred stimulating agent is phorbol 12-myristate 13-acetate, PMA.

The terms "unstimulated neutrophilic granulocytes", "unstimulated neutrophils" or "unstimulated PMNs" relates to naive vertebrate neutrophilic granulocytes, in particular to naive human neutrophilic granulocytes. The term "non-activated" is used synonymously for "unstimulated".

The terms "stimulated netotic but not netting polymorphonuclear neutrophils", "stimulated netotic but not netting PMNs", or "stimulated netotic but not netting nucleophils" relates to neutrophilic granulocytes, which are in a stimulated state and are undergoing NETosis, but without releasing NETs. Thus, stimulated netotic but not netting polymorphonuclear neutrophils undergo the changes associated with NETosis described above, and are thus "netotic", but do not undergo the final state of NETosis, i.e. rupture of the plasma membrane and the release of NETs. Neutrophils forming NETs are also called "netting" neutrophils. The term "netotic nucleophils", "NETotic nucleophils" or "NETotic PMNs" may also be used here as short term to designate stimulated netotic but not netting polymorphonuclear neutrophils, i.e. neutrophils being in a state of NETosis, but prevented from forming NETs.

The term "NET release inhibiting compound" relates to any compound inhibiting the release of NETs by stimulated neutrophils. The term thus refers to a compound aiding in producing stimulated netotic but not netting polymorphonuclear neutrophils, i.e. a compound that does not prevent that stimulated neutrophils undergo NETosis, but does essentially prevent formation or release of NETs by the stimulated neutrophils. The term in particular relates to a neutrophil serine protease inhibitor, e.g. a neutrophil elastase inhibitor. An example for such a compound is diisopropylfluorophosphate (DFP).

A "body fluid sample" means a biological sample, which consists of or comprises a body fluid, in particular a vertebrate body fluid or human body fluid. In a suitable manner, for example with buffer solution, diluted samples of body fluids are also covered by the term. Examples of body fluid samples are blood serum samples, briefly serum samples, or blood plasma samples, in short plasma samples. In particular, it is a body fluid sample containing potentially anti-neutrophil antibody. Preferably, it is a human body fluid sample. The term "aliquot of a body fluid sample" means part of a body fluid sample. Reference to a "first", "second", "third" or further aliquot, as used herein, is not to be construed as meaning a specific ranking, order or sequence of the aliquots. The numerals are only used for designating separate aliquots of a sample in order to distinguish them from each other. Further, reference to e.g. a "third aliquot" does not necessarily mean that, in each case, three aliquots have to be present. A wording like "treating a first aliquot and a third aliquot" thus means treating two aliquots, one of which is denoted "aliquot 1", the other "aliquot 3", without including treatment of a second aliquot".

The term "fixed" here has the meaning used in histology, and refers to the preservation of cells, tissue and the like for the purpose of subsequent studies. The term encompasses the immobilization of cells on a suitable carrier, e.g. a microscope slide.

The term "Neutrophil Extracellular Traps" or shortly "NETs" relates to lattices of extracellular DNA filaments containing histones and enzymes from neutrophil cytoplasmic granules (V. Brinkmann et al., 2004, Neutrophil extracellular traps kill bacteria, Science 303, 1532-1535), NETs are released by stimulated neutrophils and display diverse functions in host defence against infection, sterile inflammatory diseases, and autoimmunity (O. E. Sorensen, N. Borregaard, 2016, Neutrophil extracellular traps - the dark side of neutrophils, The Journal of clinical investigation 126, 1612-1620). Neutrophils form NETs by a multi-step cell death program (NETosis), which involves the breakdown of internal membranes, karyolysis, and the rupture of the plasma membrane (T. A. Fuchs et al., 2007, Novel cell death program leads to neutrophil extracellular traps, Journal of Cell Biology 176, 231-241; V. Papayannopoulos, K. D. Metzler, A. Hakkim, A. Zychlinsky, 2010, Neutrophil elastase and myeloperoxidase regulate the formation of neutrophil extracellular traps, Journal of Cell Biology 191, 677-691.

The term "immunodiagnostic detection" means a detection which is based on or includes an antigen-antibody binding. For example, an antigen may be detected directly by binding a labeled primary antibody to it. An indirect detection can be carried out by contacting the antigen with an antigen-binding primary antibody and subsequently binding a suitably labeled secondary antibody directed against the primary antibody to the complex of the primary antibody and the antigen. The label may be for example a fluorescent label. Suitable antibodies as well as labeling agents and methods are known in the art.

The term "Immunolabeling of anti-neutrophil antibodies" means labeling anti-neutrophil antibodies (potentially) present in the sample via direct or indirect antigen-antibody interaction. The term "immunostaining", when used herein, relates to the staining of a sample by way of one ore more labeled antibodies.

The term "ANCA-associated vasculitides (AAV)" relates to autoimmune diseases of small blood vessels in which ANCA are involved. AAV are particularly eosinophilic granulomatosis with polyangiitis (EGPA, formerly Churg-Strauss syndrome), granulomatosis with polyangiitis (GPA, formerly Wegener's disease) and microscopic polyangiitis (MPA).

The term "contacting an aliquot of a sample of body fluid with stimulated netotic but not netting polymorphonuclear neutrophils" means in particular that an aliquot of a body fluid sample is brought into contact with neutrophils, wherein the proportion of stimulated netotic but not netting neutrophils to the total number of neutrophils (including naive and netting neutrophils) is at least 1%, preferably at least 2%, 3%, 4% or at least 5%, more preferably at least 10%, 15%, 20%, 25%, 30%, 35%, 40%, 45% or 50%, particularly preferably at least 60%, 70%, 80% or at least 90%.

The term "treating stimulated, but not netting, polymorphonuclear neutrophils with a NET release inhibiting compound under conditions where the formation of NETs by the stimulated polymorphonuclear neutrophils is suppressed" is understood here to mean conditions, e.g. regarding the concentration of the NET release inhibiting compound, temperature, medium used, CO₂ content, pH etc, under which the formation of NETs is essentially suppressed, "essentially suppressed" meaning that the proportion of netting, i.e. NET releasing, stimulated neutrophils to the total number of stimulated (including netting) neutrophils is preferably below 50%, preferably below 40%, 35%, 30% or 25%, more preferably below 20%, 15% or 10%, particularly preferably below 9%, 8%, 7%, 6%, 5%, 4%, 3%, 2% or 1%. Suitable conditions are, for example, incubation of stimulated PMNs at 37 °C, 5 % CO₂, and a concentration of diisopropylfluorophosphate, DFP, of ≥ 2 mM, e.g. 2-20 mM, preferably 5-20 mM, preferably 10-20 mM or 15-20 mM, e.g. at a DFP concentration of 20 mM.

The term "contacting an aliquot of a body fluid sample with Neutrophil Extracellular Traps, NETs, produced by stimulated neutrophils" means that an aliquot of a body fluid sample is contacted with NETs, which were formed by stimulated neutrophils. The term includes both contacting an aliquot of a body fluid sample with isolated NETs, i.e. NETs separated and preferably purified from remaining cellular components, and contacting an aliquot of a body fluid sample with NETs in the presence of cells or cell bodies that have formed the NETs, or in the presence of stimulated cells that have not (yet) released any NETs.

The terms "serine protease" or "serine endopeptidase" relate to a class of proteolytic enzymes containing, at the active site, a nucleophilic serine residue involved in catalysis. The terms "neutrophil serine protease", NSP, or "neutrophil-specific serine protease" refer to a serine protease specific for neutrophils. An example for a neutrophil-specific serine protease is neutrophil elastase (NE, EC 3.4.21.37). The term "neutrophil elastase" relates in particular to human neutrophil elastase and comprises any isoform of the enzyme.

The term "serine protease inhibitor" as used herein refers to a class of compounds that interferes with the function of a serine protease. The term "neutrophil serine protease inhibitor" refers to a class of compounds that interferes with the function of a neutrophil-specific serine protease, e.g. neutrophil elastase. A compound may, for example, considered a serine protease inhibitor or neutrophil-specific serine protease inhibitor if the compound exhibits an IC50 of ≤1000 µM with at least one (neutrophil-specific) serine protease of humans or other species, preferably an IC50 of ≤800 µM, ≤500 µM, ≤250 µM, ≤100 µM, ≤90 µM, ≤80, ≤70, ≤60 or ≤50 µM, and especially preferred an IC50 of ≤40, ≤30, ≤20, ≤10 or 5 µM. Examples of serine protease inhibitors are diisopropylfluorophosphate (DFP) and phenylmethylsulfonyl fluoride (PMSF).

"Diisopropylfluorophosphate" (DFP, sometimes also abbreviated DIPF) is a compound of the formula C₆H₁₄FO₃P (CAS Number 55-91-4). DFP is a serine protease inhibitor.

The term "nuclease" relates to an enzyme cleaving phosphodiester bonds between the nucleotide subunits of nucleic acids. Examples are micrococcal nuclease (MNase, EC 3.1.31.1), DNase, e.g. DNaseI and DNase1L3. The term "DNase" refers to deoxyribonucleases, i.e. enzymes catalyzing the hydrolytic cleavage of phosphodiester linkages in the DNA backbone, thus degrading DNA. An example is DNase I (also DNase 1, E.C. 3.1.21.1), a non-specific endonuclease that degrades double- and single-stranded DNA and chromatin. A further example is DNase I like 3 (DNase1L3, DNase gamma, EC 3.1.21.-).

The term "object slide" is understood here to mean any support or carrier on or in which an examination material, in particular a biological sample, can be placed. It can, for example, be a rectangular glass object support in the form of a glass plate, as is generally used for example in microscopy or histology. Such an object slide may have the conventional dimensions (26 x 76 mm x 1-1.5 mm) in accordance with ISO 8037-1, but may also have different dimensions, for example the dimensions of a microscopic cover glass (see ISO 8255-1), glass beads or the like. Further, an object slide may, for example, be a plastic object support, a microwell plate, a membrane or suchlike, provided that the supports have a sufficient stability.

The terms "manually" or "automatically" in relation to the evaluation of staining patterns mean evaluation by hand or visually by an appropriately trained person, or by a device being designed and equipped to perform the evaluation steps. Such a device may, for example be an apparatus being able to analyse digital pictures taken from staining patterns for evaluating differences in the staining patterns.

The terms "negative control" and "positive control" have the meaning known in the art and relate to samples with known characteristics. A negative control thus, for example, represents a sample for which no response is expected, e.g. a known ANCA negative blood serum sample, whereas a positive control represents a sample for which a known response is expected, e.g. a known ANCA positive blood serum sample.

The terms "digital image of a staining pattern" or "digital image of an immunostained aliquot of a body fluid sample" refers to a digital image taken from (part of) a body fluid sample, which has been contacted with PMNs and/or NETs, e.g. on an object slide, and subsequently subjected to immunostaining, for example, with fluorescent primary or secondary antibodies. The term "digital image" also encompasses a digitized analogue picture.

In the present invention a sample of body fluid, e.g. a blood sample, or an aliquot thereof, is contacted with stimulated netotic but not netting polymorphonuclear neutrophils, PMNs, under conditions allowing binding of anti-neutrophil antibodies in the sample to the polymorphonuclear neutrophils. The netotic but not netting polymorphonuclear neutrophils have been generated by treating stimulated, but not netting, polymorphonuclear neutrophils with a NET release inhibiting compound, for example diisopropylfluorophosphate, DFP, under conditions where the formation of NETs by the stimulated polymorphonuclear neutrophils is suppressed. Subsequently, anti-neutrophil antibodies bound to the stimulated netotic but not netting polymorphonuclear neutrophils are immunolabeled, e.g. via fluorescence-labeled antibodies. The resulting staining (fluorescence) pattern of the cells stained in the body fluid sample can subsequently be evaluated manually or by means of an automated procedure. With p-ANCA positive sera stimulated netotic but not netting PMNs show a nuclear staining pattern, whereas c-ANCA positive sera show a cytoplasmic staining pattern, e.g. a bright cytoplasmic dot. The intracellular staining patterns are clearly distinguishable. Comparison of the staining patterns therefore allows for a differential diagnostic of c- and p-ANCAs. It is, of course, preferred to compare the staining patterns obtained with staining patterns of suitable negative and positive controls, e.g. with staining patterns of known c- and/or p-ANCA positive body fluid samples with stimulated netotic but not netting PMNs or with naïve formalin-fixed PMNs (positive control), and/or with staining patterns of known c- and/or p-ANCA negative body fluid samples with stimulated netotic but not netting PMNs or with naïve formalin-fixed PMNs (negative control).

A preferred NET release inhibiting compound is a compound inhibiting the release of NETs without inhibiting the translocation of MPO from the cytoplasm to the nucleus during NETosis. A suitable NET release inhibiting compound may, for example, be a serine protease inhibitor, in particular a nucleophil serine protease inhibitor (NSP inhibitor), e.g. a nucleophil elastase (NE) inhibitor like diisopropylfluorophosphate (DFP).

In the method of the invention it is preferred that the stimulated polymorphonuclear neutrophils have been generated by treating naïve polymorphonuclear neutrophils with Phorbol 12-myristate 13-acetate, PMA, under conditions where the naïve polymorphonuclear neutrophils are stimulated. Suitable conditions for stimulating naïve PMNs are, for example, incubating naïve PMNs with 0.1 µM PMA over a period of at least 30 minutes at 37 °C. However, naïve polymorphonuclear neutrophils may also be stimulated by treating them with other stimulating agents, or by exposing them to another stimulus known to have a stimulating effect, e.g. contact with artificial surfaces.

It is especially preferred that an essentially homogenous population of stimulated netotic but not netting PMNs is used in the method of the invention. Under an "essentially homogeneous population of stimulated netotic but not netting PMNs" is understood a population of PMNs, which are in essentially the same advanced state of NETosis, without, however having formed NETs. In particular, the term means that at least 75% or 80%, preferably at least 85% or 90%, especially preferred at least 91%, 92%, 93%, 94% or at least 95% of the PMNs are in essentially the same advanced state of NETosis without having formed NETs. The term may, for example, also mean a population generated by incubating naive PMNs in the presence of 0.1 µM PMA and 2 to 20 mM DFP over a period of up to 15 hours at 37°C.

In a preferred embodiment the method of the invention further involves the treatment of stimulated netotic but not netting PMNs with a nuclease, e.g. a deoxyribonuclease (DNase), preferably DNase I, DNase1-like 3 or micrococcal nuclease (MNase), prior to contacting them with an aliquot of a body fluid sample and immunolabeling anti-neutrophil antibodies bound to the stimulated netotic but not netting PMNs. The nuclease treatment removes nuclei of stimulated netotic but not netting neutrophils, and NETs. The treatment with nuclease has no effect on the binding of c-ANCAs to stimulated netotic but not netting neutrophils. However, after nuclease treatment, p-ANCAs show a pattern identical to c-ANCAs with stimulated netotic but not netting neutrophils, whereas ANA do not stain nuclease-treated nuclei of the stimulated netotic but not netting neutrophils. Therefore, nuclease treatment of stimulated netotic but not netting PMNs provides a means for reliably discriminating between p-ANCAs and ANAs.

Treatment with nuclease can, for example, mean incubating stimulated netotic but not netting PMNs, preferably fixed PMNs, with 10 U/ml DNase, e.g. DNase I, at 37°C for 30 minutes. Cells may be permeabilized before nuclease treatment. Stimulated netotic but not netting neutrophils may be fixed on an object slide and subsequently be treated with a nuclease under suitable conditions, after which step the nuclease-treated neutrophils are immunolabeled with suitable labeled antibodies, following, if necessary, possible washing steps.

In a preferred embodiment of the invention, the method of the invention thus further comprises the following steps:
b1) Contacting a further aliquot of the sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound, e.g. diisopropylfluorophosphate, DFP, under conditions where the formation of NETs by the stimulated polymorphonuclear neutrophils is suppressed, and wherein the stimulated netotic but not netting polymorphonuclear neutrophils have been incubated with a nuclease, preferably a DNase, preferably DNase1L3 or DNase I, and
b2) Immunolabeling of anti-neutrophil antibodies bound to the stimulated nuclease-treated netotic but not netting polymorphonuclear neutrophils, and/or
c1) Contacting a further aliquot of the sample of body fluid with Neutrophil Extracellular Traps, NETs, formed by stimulated polymorphonuclear neutrophils, PMNs, and
c1) Immunolabeling anti-neutrophil antibodies bound to the NETs, and/or
d1) Contacting a further aliquot of the sample of body fluid with unstimulated formalin-fixed polymorphonuclear neutrophils, PMNs, under conditions allowing binding of anti-neutrophil antibodies in the sample to the unstimulated formalin-fixed polymorphonuclear neutrophils, and
d2) Immunolabeling of anti-neutrophil antibodies bound to the unstimulated formalin-fixed polymorphonuclear neutrophils.

The inventors have found that p-ANCAs bind to NETs not treated with nuclease, thus leading to a staining of NETs, whereas c-ANCAs do not bind to NETs. Without wishing to be bound by theory, it is believed that NETs do contain MPO, but do essentially not contain PR3. Staining of NETs formed by stimulated PMNs which have not been exposed to a NET release inhibiting compound, is therefore advantageous, for example in order to confirm findings with stimulated netotic but not netting neutrophils (NETotic PMNs), or in order to alleviate automatic analysis of staining patterns. Since NETs provide a comparatively large target and p-ANCAs bind to NETs in contrast to c-ANCAs, staining of NETs is especially beneficial for distinguishing p-ANCAs and c-ANCAs, e.g. in an automated procedure.

Interestingly, the staining patterns generated by c-ANCAs and p-ANCAs are essentially the same with both nuclease-treated stimulated netotic but not netting PMNs and NETs. It is therefore not possible to distinguish between c-ANCAs and p-ANCAs by using nuclease-treated stimulated netotic but not netting PMNs or NETs alone. In addition, nuclease treatment of NETs gives essentially the same staining pattern for p-ANCAs, c-ANCAs and ANAs. Nuclease-treated NETs are therefore not useful for distinguishing p-ANCAs, c-ANCAs and ANAs. However, since ANAs neither stain stimulated netotic but not netting PMNs nor NETs, whereas c-ANCAs and p-ANCAs stain nuclease-treated stimulated netotic but not netting PMNs, a clear distinction can be made between c-ANCAs or p-ANCAs and ANAs. Since, at least in a manual procedure, a distinction between c-ANCAs and p-ANCAs can already easily and reliably be made by comparing the different intracellular staining patterns of stimulated netotic but not netting PMNs, which have not been treated with nuclease, the additional difference in the staining pattern of c-ANCAs or p-ANCAs versus the staining pattern of ANAs in nuclease-treated stimulated netotic but not netting PMNs is sufficient for a differential diagnosis between c-ANCAs, p-ANCAs and ANAs.

Evaluating the staining of NETs is especially useful in an automated procedure. The staining of NETs, which cover a large area and show only low circularity, can easily be distinguished from the staining of intact PMNs, which covers a comparatively small area and shows high circularity due to the essentially circular shape of the intact cells. This marked difference in staining pattern facilitates automatic evaluation. In an automated procedure, pictures taken from a staining pattern, e.g. on an object slide, can be processed and analysed by means of an automatic image analysis system, comparing, for example, staining areas and circularities of staining patterns. Alternatively or additionally it is also possible to perform a quantitative analysis of the observed fluorescence.

NETs formed by stimulated PMNs, whose netting has not been suppressed, preferably purified NETs, may additionally be contacted with an aliquot of the sample of body fluid, and ANCAs binding to the NETs may subsequently be immunolabeled. As mentioned above, p-ANCAs bind to NETs, thus leading to a staining of NETs in the method of the invention, whereas c-ANCAs do not bind to NETs. This may be useful to confirm the findings of a test with the netotic but not netting PMNs, in particular for the differential diagnosis of c-ANCAs and p-ANCAs. Further, this may be useful for automation of the inventive method. For the formation of NETs, naïve PMNs may be stimulated in a suitable manner in the absence of a NET release inhibiting compound like DFP, e.g. by incubating them in the presence of 0.1 µM PMA over a period of, for example, 4 hours at 37°C.

An aliquot of the sample of body fluid could thus for example be contacted with stimulated netotic but not netting PMNs, and in parallel with NETs formed by stimulated PMNs. The stimulated netotic but not netting PMNs and NETs may, for example, be arranged on different object slides, or in different areas of the same object slide. Preferably, the PMNs and NETs have previously been fixed on the object slide(s). Further preferred, the PMNs and NETs have initially, i.e. preferably prior to stimulation, been purified. NETs may have been further purified by eliminating cell debris. Subsequent immunolabeling of the netotic but not netting PMNs and NETs will result in staining patterns, which can be compared manually or automatically. A p-ANCA positive sample, e.g. blood serum sample, would, for example, result in a staining of the nuclei of the stimulated netotic but not netting PMNs and the NETs, whereas a c-ANCA positive sample would result in a cytoplasmic staining of the stimulated netotic but not netting PMNs and no staining of the NETs. While it is possible for an automated procedure to automatically distinguish between the nuclear staining of p-ANCAs and the cytoplasmic staining of c-ANCAs in stimulated netotic but not netting PMNs, the different stainings of NETs by p- and c-ANCAs can more easily be automatically compared. Analyzing staining patterns of NETs released by PMNs is therefore especially preferred in case of an automation of the method of the invention.

Further, since p-ANCAs, but not ANAs, stain formalin-fixed unstimulated PMNs, an aliquot of the body fluid sample may also be contacted with such formalin-fixed unstimulated PMNs, for example to confirm the findings with nuclease-treated NETotic PMNs.

Examples of useful stainings or staining combinations are given in Table 1. It is to be noted that suitable controls are not mentioned for reasons of better overview.

**Table 1: Examples of possible staining combinations. The term "NETotic PMNs" is used here for stimulated netotic but not netting PMNs.**

| No. | Staining (combination) | Discrimination between cANCAs and pANCAs | Discrimination between pANCAs and ANAs |
|---|---|---|---|
| 1 | NETotic PMNs | + | - |
| 2 | NETotic PMNs, nuclease treated | - | + |
| 3 | NETotic PMNs plus NETs | + | - |
| 4 | NETotic PMNs plus NETotic PMNs, nuclease treated | + | + |
| 5 | NETotic PMNs, nuclease treated, plus NETs | + | + |
| 6 | NETotic PMNs plus NETotic PMNs, nuclease treated plus NETs | + | + |
| 7 | NETotic PMNs plus unstimulated neutrophils (formalin-fixed) | + | + |
| 8 | NETotic PMNs plus NETs | | |
| | plus unstimulated neutrophils (formalin-fixed) | + | + |

For example, an embodiment of the method of the invention according to combination 3 of table 1 may thus comprise the following steps:
a1) Contacting an aliquot of a sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound, e.g. diisopropylfluorophosphate, DFP, under conditions where the formation of NETs by the stimulated polymorphonuclear neutrophils is suppressed, and
a2) Immunolabeling of anti-neutrophil antibodies bound to the stimulated netotic but not netting polymorphonuclear neutrophils; and
d1) Contacting a further aliquot of the sample of body fluid with Neutrophil Extracellular Traps, NETs, formed by stimulated polymorphonuclear neutrophils, PMNs, under conditions allowing binding of anti-neutrophil antibodies in the sample to the NETs, and
d2) Immunolabeling of anti-neutrophil antibodies bound to the NETs.

This staining combination allows discrimination between p-ANCAs and c-ANCAs, but not between p-ANCAs and ANAs. The PMNs/NETs may e.g. be arranged on separate object slides or in different areas of the same object slide

For a further discrimination between p-ANCA and ANA, an aliquot of the body fluid sample may in parallel be contacted with DNase-treated stimulated netotic but not netting PMNs (see e.g. combination no. 6). The PMNs/NETs may e.g. be arranged on separate object slides or in different areas of the same object slide.

Thus, the method of the invention in a further preferred embodiment comprises:
a1) Contacting a first aliquot of a sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound, for example diisopropylfluorophosphate, DFP, under conditions where the formation of NETs by the stimulated polymorphonuclear neutrophils is suppressed, and
a2) Immunolabeling of anti-neutrophil antibodies bound to the stimulated netotic but not netting polymorphonuclear neutrophils; and
b1) Contacting a second aliquot of the sample of body fluid with stimulated netotic but not netting polymorphonuclear neutrophils, PMNs, under conditions allowing binding of anti- neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound, for example diisopropylfluorophosphate (DFP), under conditions where the formation of NETs by the stimulated polymorphonuclear neutrophils is suppressed, and wherein the stimulated netotic but not netting polymorphonuclear neutrophils have been treated with a nuclease, preferably a DNase, preferably DNase I, and
b2) Immunolabeling of anti-neutrophil antibodies bound to the stimulated nuclease-treated netotic but not netting polymorphonuclear neutrophils, and
c1) Contacting a third aliquot of the sample of body fluid with Neutrophil Extracellular Traps, NETs, formed by stimulated polymorphonuclear neutrophils, PMNs, under conditions allowing binding of anti-neutrophil antibodies in the sample to the NETs, and
c2) Immunolabeling anti-neutrophil antibodies bound to the NETs.

In another embodiment of a preferably automated method of the invention NETs formed by stimulated PMNs not treated with a NET release inhibiting compound or a nuclease, and nuclease-treated stimulated netotic but not netting PMNs are immunostained and the staining patterns obtained are compared. In this embodiment, the method of the invention thus comprises:
b1) Contacting a first aliquot of the sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound, for example diisopropylfluorophosphate, DFP, under conditions where the formation of NETs by the stimulated polymorphonuclear neutrophils is suppressed, and wherein the stimulated netotic but not netting polymorphonuclear neutrophils have been treated with a nuclease, preferably a DNase, further preferred DNase1L3 or DNase I, and
b2) Immunolabeling of anti-neutrophil antibodies bound to the stimulated DNase-treated netotic but not netting polymorphonuclear neutrophils, and
d1) Contacting a second aliquot of the sample of body fluid with Neutrophil Extracellular Traps, NETs, formed by stimulated polymorphonuclear neutrophils, PMNs, under conditions allowing binding of anti-neutrophil antibodies in the sample to the NETs, and
d2) Immunolabeling anti-neutrophil antibodies bound to the NETs.

In the method of the invention staining patterns obtained for different aliquots of the sample of body fluid are preferably compared with each other and with staining patterns obtained for a negative and/or a positive control, if and insofar as suitable and beneficial, in particular for a differential ANCA diagnosis. The method of the invention therefore preferably comprises
i) comparing the staining pattern obtained in step a2 with staining patterns obtained for a negative and/or a positive control, or
ii) comparing the staining patterns obtained in steps a2 and b2 with each other and with staining patterns obtained for a negative and/or a positive control, or
iii) comparing the staining patterns obtained in steps a2, b2 and c2 with each other and with staining patterns obtained for a negative and/or a positive control, or
iv) comparing the staining patterns obtained in steps a2 and c2with each other and with staining patterns obtained for a negative and/or a positive control, or
v) comparing the staining patterns obtained in steps a2, b2, c2 and d2 with each other and with staining patterns obtained for a negative and/or a positive control, or
vi) comparing the staining patterns obtained in steps a2, b2 and d2 with each other and with staining patterns obtained for a negative and/or a positive control, or
vii) comparing the staining patterns obtained in steps a2 and d2 with each other and with staining patterns obtained for a negative and/or a positive control.

The method according to the first aspect of the invention can be largely or even fully automated. The clear and distinctive staining patterns obtained can be automatically evaluated, e.g. by means of an image recognition and analysis system capturing and analysing digital images taken from the staining patterns.

In a preferred embodiment, wherein the method of the invention is at least partially carried out automatically, the method therefore comprises:
i) preparing digital images of the staining pattern obtained in step a2 and of the staining patterns of the negative and/or positive controls, and determining and comparing the area and the circularity of the staining patterns obtained in step a2 and of the negative and/or positive controls, or
ii) preparing digital images of the staining patterns obtained in steps a2 and b2 and of the staining patterns of the negative and/or positive controls, and determining and comparing the area and the circularity of the staining patterns obtained in steps a2 and b2, and of the negative and/or positive controls, or
iii) preparing digital images of the staining patterns obtained in steps a2, b2 and c2, and of the staining pattern of the negative and/or positive control, and determining and comparing the area and the circularity of the staining patterns obtained in steps a2, b2 and c2, and of the negative and/or positive controls, or
iv) preparing digital images of the staining patterns obtained in steps a2 and c2, and of the staining patterns of the negative and/or positive controls, and determining and comparing the area and the circularity of the staining patterns obtained in steps a2 and c2, and of the negative and/or positive controls, or
v) preparing digital images of the staining patterns obtained in steps a2, b2, c2 and d2, and of the staining patterns of the negative and/or positive controls, and determining and comparing the area and the circularity of the staining patterns obtained in steps a2, b2, c2 and d2, and of the negative and/or positive controls, or
vi) preparing digital images of the staining patterns obtained in steps a2, b2 and d2, and of the staining patterns of the negative and/or positive controls, and determining and comparing the area and the circularity of the staining patterns obtained in steps a2, b2 and d2, and of the negative and/or positive controls, or
vi) preparing digital images of the staining patterns obtained in steps a2 and d2, and of the staining patterns of the negative and/or positive controls, and determining and comparing the area and the circularity of the staining patterns obtained in steps a2 and d2, and of the negative and/or positive controls.

The method according to the fourth aspect is especially useful for the (partially) automatic differential diagnostic analysis of c-ANCA and p-ANCA, or of c-ANCA, p-ANCA and ANA in body fluid samples.

In a preferred embodiment binary black and white images of the immunostained sections are compared.

The method makes use of the marked differences in the staining patterns obtained with stimulated netotic but not netting PMNs and NETs formed by PMNs, and allows for a relatively simple, reliable and automated ANCA diagnosis. Although the circularity of p-ANCA staining patterns and c-ANCA staining patterns on NETotic neutrophils are generally not different, p-ANCA staining patterns on NETotic neutrophils are generally larger in area than C-ANCA staining pattern, and can thus also be automatically distinguished using a suitable algorithm. The differences in the staining patterns obtained after nuclease treatment of stimulated netotic but not netting PMNs permit discrimination between, for example, p-ANCAs and ANAs without the need for performing separate tests with naive PMNs or an ELISA.

In an embodiment of the method according to the first aspect of the invention, for example, colored microscopic images initially taken from immunostained fixed stimulated netotic but not netting, optionally nuclease-treated, PMNs and/or NETs, are converted to a compressed digital image format, for example, JPEG, and to 8-bit grayscale images. The 8-bit grayscale images can be converted to binary (1-bit) black and white images by using an appropriate threshold value, thus obtaining black areas and outlines. The circularity, i.e. the degree of circularity, and the area of stained sections can then be determined and compared. If, for example, predominantly NETs were stained, e.g. by p-ANCA, the resulting image shows a relatively low degree of circularity, and a comparatively large area of stained structures. In the case of, for example stimulated netotic but not netting PMNs contacted with c-ANCA in the body fluid sample, the image would, on the other hand, give a comparatively large circularity and small area of stained structures. These measures can all be performed automatically by means of suitable and available image analysis programs.

Preferably, the immunolabeling of antibodies bound to the stimulated netotic but not netting PMNs or to NETs, irrespective of whether or not the PMNs or NETs have been treated with a nuclease beforehand, is done by means of fluorescently labeled antibodies. Anti-IgG antibodies known to the skilled person may be used, preferably anti-hIgG antibodies suitably labeled with a fluorescent dye. Such antibodies are known and commercially available. Suitable are, for example primary and/or secondary antibodies used in the ANCA-diagnostics known in the art.

Stimulated netotic but not netting PMNs or NETs are preferably purified in a suitable manner before contacting them with the sample of body fluid. For generating purified stimulated netotic but not netting polymorphonuclear neutrophils naive neutrophils can be purified and subsequently be stimulated in vitro. Purified NETs can be obtained from purified stimulated PMNs, and can additionally be purified in order to free them from cell debris, if necessary or desired. Suitable purification methods are known to the skilled person and, for example, described in Nauseef et al. 2007, Isolation of Human Neutrophils From Venous Blood, Methods in Molecular Biology 412, 15-20.

Preferably, but not necessarily, the body fluid sample is contacted with stimulated netotic but not netting neutrophils or with NETs, if applicable, which are fixed in a suitable manner, for example, ethanol or formalin fixed. Preferably, the stimulated neutrophils or NETs are purified prior to fixation. Suitable fixation methods are known to the skilled person and described for example in Yang P. 1997, Comparative evaluation of unfixed and fixed human neutrophils for determination of antineutrophil cytoplasmic antibodies by indirect immunofluorescence, Journal of Clinical Pathology 50, 677-80. Particularly preferred the stimulated netotic but not netting neutrophils or NETs, if applicable, are immobilized on a suitable carrier. Suitable carriers are, for example, glass slides, beads, e.g. glass or plastic beads, and the like.

In a second aspect the present invention provides a method for preparing an object slide for the immunodiagnostic detection of anti-neutrophil antibodies, the method comprising:
a) Generating stimulated netotic but not netting polymorphonuclear neutrophils, PMNs, by treating stimulated polymorphonuclear neutrophils, PMNs, with a NET release inhibiting compound under conditions where the formation of NETs by the stimulated polymorphonuclear neutrophils is suppressed, and
b) Fixing the stimulated netotic but not netting PMNs on an object slide.

A preferred NET release inhibiting compound is a compound inhibiting the release of NETs without inhibiting the translocation of MPO from the cytoplasm to the nucleus during NETosis. A suitable NET release inhibiting compound may, for example, be a serine protease inhibitor, in particular a nucleophil serine protease inhibitor (NSP inhibitor), e.g. a nucleophil elastase (NE) inhibitor like diisopropylfluorophosphate (DFP).

The stimulated PMNs may be generated by exposing naive PMNs to a stimulus inducing NETosis, e.g. a chemical compound or another stimulus, such as contact to an artificial surface, causing the naive PMNs to enter NETosis. A preferred stimulating chemical compound is phorbol 12-myristate 13-acetate, PMA. Naive PMNs may, for example, be stimulated in the presence of 0.1 µM PMA over a period of at least 30 minutes at 37 °C.

In a preferred embodiment of the method naive PMNs are stimulated in the presence of the NET release inhibiting compound, e.g. DFP, under conditions that an essentially homogenous population of stimulated netotic but not netting PMNs is obtained. This may, for example be achieved by contacting naive polymorphonuclear neutrophils with phorbol 12-myristate 13-acetate, PMA, preferably with 0.1 µM PMA in the presence of 2-20 mM DFP over a period of up to 15 hours at 37°C.

The fixation of the PMNs can be achieved by methods well known in the art. The PMNs may, for example be formalin-fixed.

In a further preferred embodiment of the method the fixed stimulated netotic but not netting PMNs are treated with a nuclease, preferably a DNase, further preferred DNase1L3 or DNase I. Preferably, the fixed stimulated netotic but not netting PMNs are permeabilized prior to treating them with nuclease.

In a further preferred embodiment of the invention, the method comprises:
Generating stimulated netting polymorphonuclear neutrophils, PMNs, and
Fixing NETs released by the stimulated netting polymorphonuclear neutrophils on an object slide.

The generated stimulated netotic but not netting PMNs and/or NETs may be fixed on different slides or different areas of the same slide.

Preferably the PMNs and NETs, if applicable, have been purified prior to stimulation. NETs may have been further purified by eliminating cell debris.

In a third aspect, the invention provides an object slide or object slide set for the immunodiagnostic detection of anti-neutrophil antibodies, the object slide or object slide set having fixed thereon:
a) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and/or
b) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease.

In a preferred embodiment the object slide or object slide set of the third aspect of the invention further comprises, fixed thereon,
c) Neutrophil Extracellular Traps, NETs, formed by stimulated polymorphonuclear neutrophils, PMNs, and/or
d) unstimulated formalin-fixed polymorphonuclear neutrophils, PMNs.

The stimulated netotic but not netting PMNs, the nuclease-treated stimulated netotic but not netting PMNs and/or the nuclease-treated NETs may be fixed on different object slides or in different areas on a single object slide.

In preferred embodiments the object slide or object slide set comprises fixed thereon:
i) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, or
ii)
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, or
iii)
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, and
   - Neutrophil Extracellular Traps, NETs, formed by stimulated polymorphonuclear neutrophils, PMNs, or
iv)
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
   - Neutrophil Extracellular Traps, NETs, formed by stimulated polymorphonuclear neutrophils, PMNs, or
v)
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, and
   - Neutrophil Extracellular Traps, NETs, formed by stimulated polymorphonuclear neutrophils, PMNs, and
   - unstimulated formalin-fixed polymorphonuclear neutrophils, PMNs, or
vi)
   - stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
   - unstimulated formalin-fixed polymorphonuclear neutrophils, PMNs.

Thus the object slide or object slide set according to the third aspect of the invention may, for example, have the following substrates or combinations of substrates fixed thereon:
i) NETotic PMNs, or
ii) NETotic PMNs plus nuclease-treated NETotic PMNs, or
iii) NETotic PMNs plus nuclease-treated NETotic PMNs plus NETs, or
iv) NETotic PMNs plus NETs, or
v) NETotic PMNs plus nuclease-treated NETotic PMNs plus NETs plus formalin-fixed unstimulated PMNs, or
vi) NETotic PMNs plus formalin-fixed unstimulated PMNs.

The term "object slide set", as used herein, refers to a combination, i.e. a plurality, of different object slides having fixed thereon different substrates or substrate combinations. As used herein, the term refers to embodiments in which different substrates, e.g. NETotic PMNs, nuclease-treated NETotic PMNs, NETs or formalin-fixed unstimulated PMNs are not combined on a single object slide but are placed on separate object slides, which, however, are combined in a set of object slides. The invention thus not only provides a single object slide having fixed thereon a combination of different substrates, but also a set of object slides, the set comprising a plurality of object slides, each having fixed thereon a single substrate or a combination of substrates which is different from the substrate or combination of substrates on each of the other object slides being part of the set.

A set of object slides may thus, for example, comprise:
- An object slide having fixed thereon NETotic PMNs plus an object slide having fixed thereon nuclease-treated NETotic PMNs, or
- An object slide having fixed thereon NETotic PMNs plus an object slide having fixed thereon nuclease-treated NETotic PMNs plus NETs, or
- An object slide having fixed thereon NETotic PMNs plus an object slide having fixed thereon NETs, or
- An object slide having fixed thereon NETotic PMNs plus an object slide having fixed thereon nuclease-treated NETotic PMNs plus an object slide having fixed thereon NETs plus an object slide having fixed thereon formalin-fixed unstimulated PMNs, or
- An object slide having fixed thereon NETotic PMNs plus an object slide having fixed thereon formalin-fixed unstimulated PMNs.

A preferred NET release inhibiting compound used for generating stimulated netotic but not netting polymorphonuclear neutrophils is a compound inhibiting the release of NETs without inhibiting the translocation of MPO from the cytoplasm to the nucleus during NETosis. A suitable NET release inhibiting compound may, for example, be a serine protease inhibitor, in particular a nucleophil serine protease inhibitor (NSP inhibitor), e.g. a nucleophil elastase (NE) inhibitor like diisopropylfluorophosphate (DFP).

The nuclease is preferably a DNase, for example DNase I or DNase1-like 3.

Stimulated PMNs may be generated by exposing naive PMNs to a stimulus inducing NETosis, e.g. a chemical compound or another stimulus, such as contact to an artificial surface, causing the naive PMNs to enter NETosis. A preferred stimulating chemical compound is phorbol 12-myristate 13-acetate, PMA. Naive PMNs may, for example, be stimulated in the presence of 0.1 µM PMA over a period of at least 30 minutes at 37 °C.

Naive PMNs are preferably be stimulated in the presence of the NET release inhibiting compound, e.g. DFP, under conditions that an essentially homogenous population of stimulated netotic but not netting PMNs is obtained. This may, for example be achieved by contacting naive polymorphonuclear neutrophils with phorbol 12-myristate 13-acetate, PMA, preferably with 0.1 µM PMA in the presence of 2-20 mM DFP over a period of up to 15 hours at 37°C. On an object slide is thus preferably provided a fixed and essentially homogenous population of stimulated netotic but not netting PMNs, which may have been treated with a nuclease, preferably after fixation.

The fixation of the PMNs can be achieved by methods well known in the art. The PMNs may, for example be formalin-fixed.

In a fourth aspect the invention relates to a kit for the immunodiagnostic detection of anti-neutrophil antibodies, the kit comprising:
a) at least one object slide or object slide set according the third aspect of the invention, and
b) at least one fluorescently labeled anti-neutrophil antibody.

Preferably, the kit of the invention additionally comprises suitable detergents and or buffer solutions. Such solutions are known in the art.

In a fifth aspect the invention relates to the use of
a) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound, preferably a serine protease inhibitor, preferably a nucleophil serine protease inhibitor, further preferred a nucleophil elastase inhibitor, and especially preferred diisopropylfluorophosphate, DFP or phenylmethylsulfonyl fluoride, PMSF, under conditions where the formation of NETs by the stimulated polymorphonuclear neutrophils is suppressed, and/or
b) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound, preferably a serine protease inhibitor, preferably a nucleophil serine protease inhibitor, further preferred a nucleophil elastase inhibitor, and especially preferred diisopropylfluorophosphate, DFP, or phenylmethylsulfonyl fluoride, PMSF under conditions where the formation of NETs by the stimulated polymorphonuclear neutrophils is suppressed, and wherein the stimulated netotic but not netting polymorphonuclear neutrophils have been treated with a nuclease, preferably a DNase, further preferred DNase1L3 or DNase I,
for the immunodiagnostic detection of anti-neutrophil antibodies, preferably in a body fluid sample, such as a plasma or serum sample from an individual afflicted with or supposed to be afflicted with an autoimmune disease, preferably an ANCA-associated vasculitis, for example, Granulomatosis with polyangiitis (GPA) or microscopic polyangiitis (MPA).

A preferred NET release inhibiting compound is a compound inhibiting the release of NETs without inhibiting the translocation of MPO from the cytoplasm to the nucleus during NETosis.

The invention is described hereinafter for illustrative purposes only in more detail with reference to the appended figures.

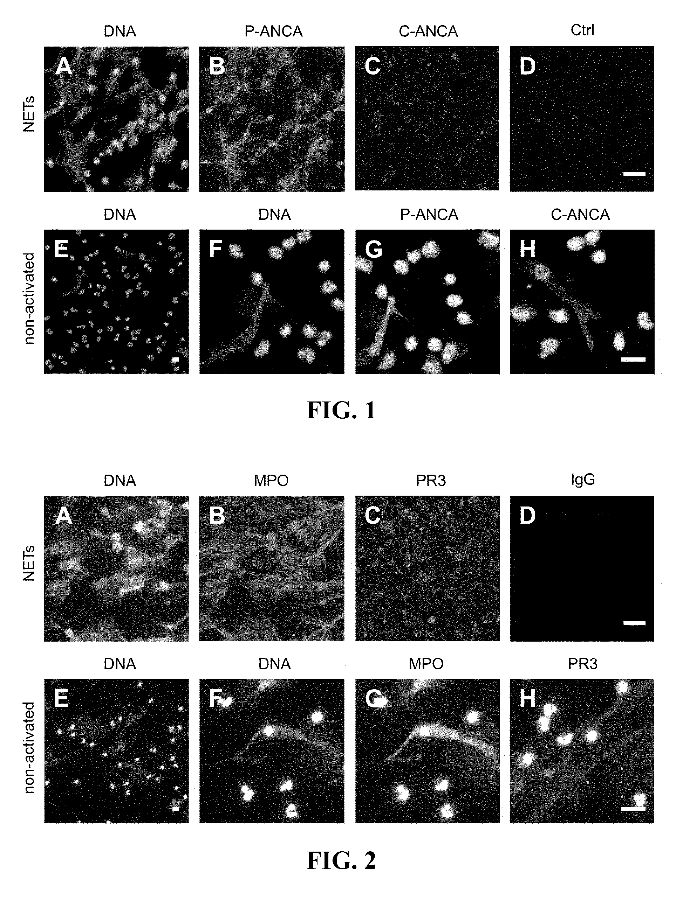

Figure 1. NETs are a substrate for the binding of P-ANCAs. (A-D) Stainings of PMA-stimulated (activated) neutrophils. Bar represents 50 µm. (A) DNA staining shows NETs as elongated fibers (B) Staining for anti-human IgG with P-ANCA positive serum shows NETs. (C) C-ANCA-positive serum does not stain NETs, but the cell bodies of netting neutrophils. (D) Staining with autoantibody-free serum (Ctrl) does not stain neutrophils or NETs. (E-H) Staining of unstimulated (non-activated) neutrophils. Bar represents 20 µm. (E) DNA staining shows nuclei of neutrophils and spontaneously formed NETs. Bar represents 20 µm. (F) Magnification of panel E. (G, H) Overlay of DNA staining and human IgG staining. (G) P-ANCA positive serum stained spontaneously formed NET along with the cytoplasm of naive cells. (H) C-ANCA positive serum stained the cytoplasm of naive cells but not spontaneously formed NET. Images are representative of 3 or more independent experiments with sera from 3 or more donors.
Figure 2. NETs are rich in myeloperoxidase, but not proteinase 3. (A-D) Stainings of PMA-activated neutrophils. Bar represents 50 µm. (A) DNA staining shows NETs as elongated fibres. (B) Staining with anti-myeloperoxidase-IgG shows NETs. (C) Anti-PR3-antibodies do not stain NETs, but label cell bodies of netting neutrophils. (D) Staining with serum from control IgG does not stain neutrophils or NETs. (E-H) Staining of unstimulated (non-activated) neutrophils. Bar represents 20 µm. (E) DNA staining shows nuclei of neutrophils and spontaneously formed NETs. Bar represents 20 µm. (F) Magnification of panel E. (G) Anti-myeloperoxidase-IgG stained spontaneously formed NETs along with the cytoplasm of naive cells. (H) Anti-proteinase 3-IgG stained the cytoplasm of naive cells but not spontaneously formed NET. Images are representative of 3 or more independent experiments.

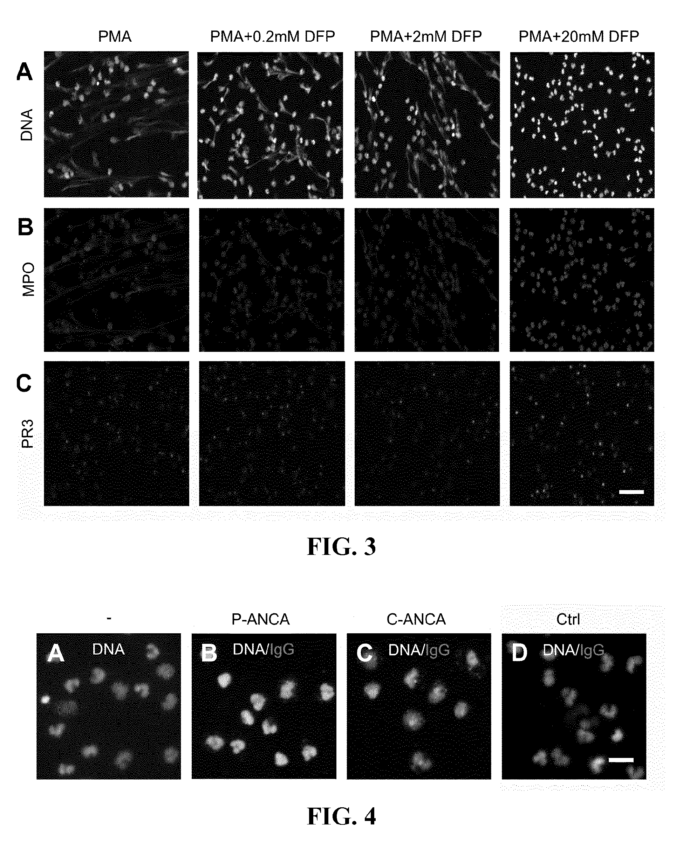

Figure 3. Protease inhibition in NETosis generates neutrophils with MPO-positive nuclei. Neutrophils were activated with PMA in the presence of indicated concentrations of the serine-protease inhibitor DFP. (A) DNA staining. DFP dose-dependently blocked the disintegration of nucleus and generation of NETs. (B) Staining for MPO. DFP dose-dependently increased the number of MPO positive nuclei. (C) Staining for PR3. DFP did not cause nuclear translocation of PR3. Scale bar represents 50 µm
Figure 4. NETotic neutrophils are substrate to discriminate ANCA-subtypes. Neutrophils were activated with PMA in the presence of 20mM DFP for 15 hours to generate a homogenous population of 'NETotic'neutrophils. (A) DNA staining shows lobulated nuclei. (B) Staining with P-ANCA positive serum shows a nuclear staining pattern in 'NETotic' neutrophils. (C) Staining with C-ANCA positive serum shows a cytoplasmic staining in 'NETotic neutrophils. (D) No staining was seen with autoantibody-negative control sera. Scale bars represent 20 µm. Images are representative of 3 or more independent experiments with sera from 3 more donors.

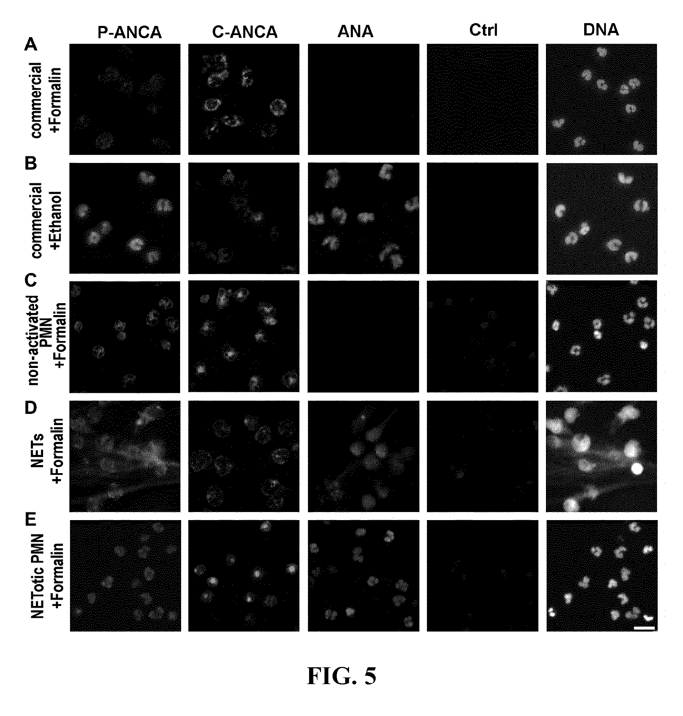

Figure 5. Ethanol-fixation mimics NETosis. Comparison of ANCA-detection by commercial substrates to NETs and NETotic neutrophils. Substrates were incubated with sera containing P-ANCAs, C-ANCAs, ANAs, or autoantibody-free control serum (Ctrl). The figure shows the staining for human IgG and DNA of P-ANCA-serum. Scale bar represent 20 µm. (A, B) Commercial ANCA-substrates. (A) Unstimulated (non-activated) ethanol-fixed neutrophils. (B) Unstimulated (non-activated) formalin-fixed neutrophils. (C-E) ANCA-substrates generated as described in Material and Methods. (C) Unstimulated (non-activated) neutrophils detect a cytoplasmic staining with C- and P-ANCAs, similar to the commercial substrate in panels A. (D) NETs bind P-ANCAs and ANAs. C-ANCAs show reactivity with the cell body of netting neutrophils. (E) NETotic neutrophils. P-ANCAs and ANAs bind the nuclei, whereas C-ANCAs bind the cytoplasm. Ethanol-fixed neutrophils show similar staining patterns as NETotic neutrophils. Autoantibody-negative control serum (Ctrl) does not stain non-activated neutrophils, NETs, or NETotic neutrophils.

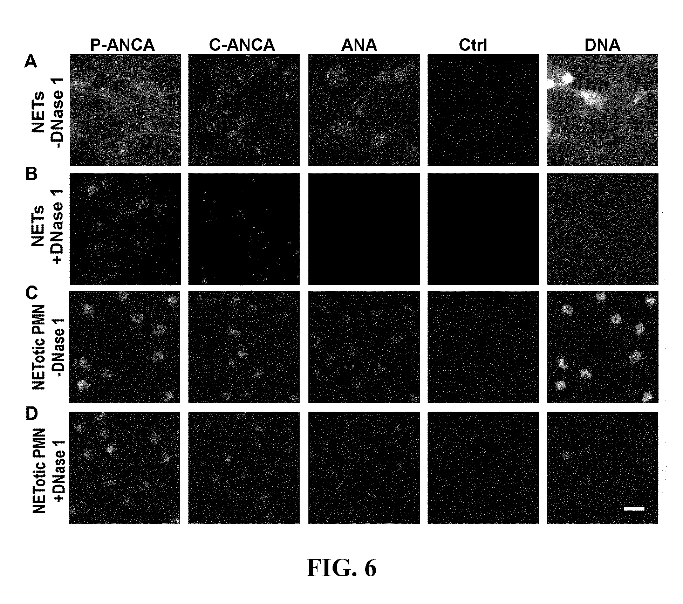

Figure 6. DNase-treatment of NETs and NETotic neutrophils differentiates ANCAs from ANAs. Analysis of ANCA- and ANA-detection by DNase1-treated NETs and NETotic neutrophils. The figure shows the staining for human IgG and DNA of substrates incubated with P-ANCA-serum. Scale bar represents 50 µm. (A) NETs without DNase1-treatment bind P-ANCAs and ANAs. (B) DNase1-treatment of NETs prevents the binding of P-ANCAs and ANAs. (C) NETotic neutrophils without DNase1-treatment give a nuclear staining with P-ANCAs and ANAs and a cytoplasmic staining with C-ANCAs. (D) NETotic neutrophils treated with DNase1 show a cytoplasmic staining with P- and C-ANCAs and do not bind ANAs.

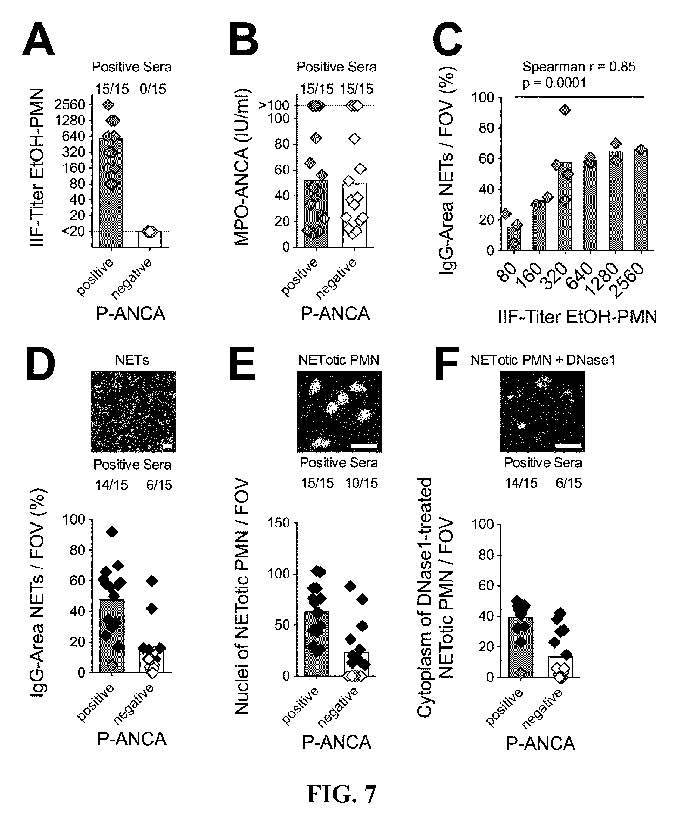

Figure 7. NETs detect MPO-ANCAs in P-ANCA-negative sera. (A) IIF-titer on ethanol-fixed neutrophils and (B) quantification of MPO-ANCA by ELISA of P-ANCA-positive (n = 15) and P-ANCA-negative sera. (C) Correlation of IIF-titer on ethanol-fixed neutrophils of P-ANCA-positive sera with IgG-staining area of NETs per field of view. (D-F) Images show characteristic staining patterns of human IgG and DNA of NETs (D, bar = 50 µm), NETotic PMN (E, bar = 20 µm), and DNase1-treated NETotic PMNs (F, bar = 20 µm). (D) A NET-staining pattern was observed with all 14/15 P-ANCA-positive sera and 6/15 P-ANCA-negative sera. Quantification of the IgG-staining area per field of view (FOV) of analyzed images. Black diamonds indicate positive sera. (E) Nuclei of NETotic neutrophils were stained with all P-ANCA-positive sera and 10 of 15 P-ANCA-negative sera. Quantification of ANCA-positive nuclei per FOV. Black diamonds indicate positive sera. (F) Cytoplasmic staining patterns on DNase1-treated NETotic neutrophils were observed with 14/15 P-ANCA-positive sera and 6 of 15 P-ANCA-negative sera. Quantification of ANCA-positive cytoplasm per FOV. Black diamonds indicate positive sera.

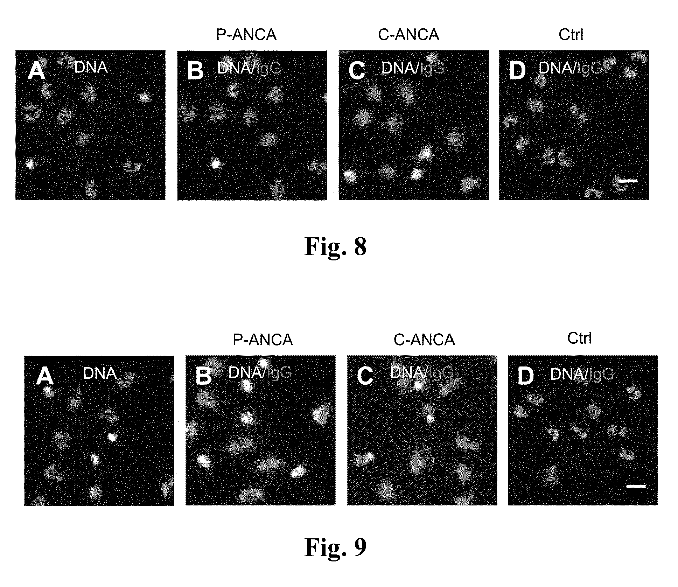

Figure 8. ANCA staining of unstimulated neutrophils. (A) DNA staining of unstimulated neutrophils showing lobulated nuclei (B) p-ANCA positive sera and (C) c-ANCA-positive sera give a cytoplasmic staining pattern on unstimulated neutrophils. (D) No staining was seen in case of autoantibody negative sera. Scale bar represents 20 µm.
Figure 9. MPO- and PR3-staining of unstimulated neutrophils. (A) DNA staining of unstimulated neutrophils showing lobulated nucleus. (B) Staining for MPO and (C) for PR3 antibody shows the cytoplasmic localization of granular enzymes. (D) No staining was observed with unspecific IgG control.

### Examples:

It is to be noted that the terms "NETotic neutrophils", "NETotic cells" or "NETotic PMNs" are used to denote stimulated netotic but not netting PMNs.

### Materials and Methods

### Neutrophil isolation

Neutrophils were isolated as described in T. A. Fuchs et al., 2007, Novel cell death program leads to neutrophil extracellular traps. Journal of Cell Biology 176, 231-241. Peripheral blood was collected from healthy volunteer donors and anticoagulated with ethylenediaminetetraacetic acid (EDTA, monovette, Sarstedt). In brief, blood was layered onto Histopaque 1119 (Sigma Aldrich). After centrifugation for 20 min at 800 g, the neutrophil-rich layer was collected. The cells were washed with Hanks-buffered salt solution without divalent cations (HBSS-, Life Technologies) supplemented with 5 mM EDTA and 0.1% bovine serum albumin (BSA, Sigma-Aldrich). Washed cells were further fractionated on a discontinuous Percoll gradient (GE Healthcare). After centrifugation for 20 min at 800 g, the neutrophil-rich layer was collected and washed with 0.1% BSA in HBSS-. All procedures were conducted at room temperature. Neutrophil viability was greater than 98%, as determined by trypan blue (Sigma-Aldrich) exclusion.

### Induction of NETosis

Isolated neutrophils were seeded onto sterile 96 well optical plates (Falcon) at a concentration of 5 x 10⁴ cells per well in Dulbecco's Modified Eagle Medium (DMEM, Gibco) medium. For non-activated cells, the naive neutrophils were seeded onto the plate and incubated for 15 minutes. To induce NET-formation, 0.1µM phorbol 12-myristate 13-acetate (PMA, Sigma-Aldrich) was added to the cells. The cells were then incubated for 4 hours. For NETotic neutrophils, diisopropylfluorophosphate (DFP, Sigma-Aldrich) was added at 20mM or indicated concentrations to the cells before addition of 0.1µM PMA and the cells were incubated for up to 15 hours. All incubations were performed at 37°C with 5% CO₂.

### Fixation of cells

Cells were fixed with 2% paraformaldehyde (PFA, Sigma Aldrich) overnight at 4°C in case of unstimulated cells and NETs. NETotic cells were fixed for 2 hours at 4°C.

### Indirect Immunofluorescence (IIF)

Prior to immunostaining, fixed neutrophils were washed twice with phosphate-buffered-saline. The cells were permeabilized with 0.5% Triton X-100 diluted in PBS for 5 minutes. For blocking, the wells were incubated with 150 µl of 2% bovine serum albumin (BSA) in PBS. Primary and secondary antibodies were used at the following concentrations: 2.5µg/ml rabbit anti-human myeloperoxidase antibody (DAKO, product number A0398), 5 µg/ml of mouse anti-human proteinase 3 antibody (WGM2, Csernok E, Ludemann J, Gross WL, & Bainton DF, 1990, Ultrastructural localization of proteinase 3, the target antigen of anti-cytoplasmic antibodies circulating in Wegener's granulomatosis. The American journal of pathology 137(5):1113-1120), 5 µg/ml of Alexa Fluor-546 goat anti-rabbit IgG (Life Technologies), 10 µg/ml of Alexa Fluor-488 goat anti-mouse IgG (Life Technologies), 10 µg/ml Alexa Fluor-555 goat-anti-human IgG (all Life Technologies), or 5 µg/ml of fluorescein(FITC)-conjugated polyclonal rabbit anti-human IgG antibody (DAKO; F0202). All the antibodies were diluted in PBS with 0.05% Tween (PBST). Human sera used for IIF were diluted 1:20 with PBST containing 5 mM EDTA, 0.1 BSA. Human sera or primary antibodies were incubated with cells at 37°C for 45 minutes. The wells were washed twice with PBST for 5 minutes before the addition of the secondary antibodies. The cells were incubated with 5 µg/ml 4',6-diamidino-2-phenylindole DAPI (Sigma-Aldrich) to counterstain the DNA. After the final wash, 50 µl of PBS was added to the cells. Fluorescent images were acquired on the day of the IIF-staining using an inverted fluorescence microscope (ZeAxiovert 200M, Zeiss).

### Commercial ANCA-detection kit and sera

A commercial kit with ethanol- and formalin fixed neutrophils was used to detect ANCA by IIF (Euroimmun, FA1201-1005-22). The following sera from Euroimmune were used: P-ANCA-positive serum (CA 1211-0105-3), C-ANCA-positive serum (CA1200-0110-3), ANA-positive serum (CA1570-0101-3), autoantibody-free serum (CA1000-0110).

### DNase 1 treatment of NETs and NETotic cells

After fixation, the cells were washed twice with phosphate-buffered saline (PBS, Life Technologies) and permeabilized with 0.5% Triton X-100 (Sigma-Aldrich). NETs or NETotic cells were treated with 10 U/ml recombinant human DNase1 (rh DNase 1, Roche) in Hanks-buffered salt solution with divalent cations (HBSS+, Life Technologies) for 30 minutes at 37°C. Thereafter, the cells were washed once with PBS.

### Results

### P-ANCAs target NETs.

To test whether ANCAs bind to NETs, NETosis was induced by activating human neutrophils for 4 hours with phorbol-12-myristate-13-acetate (PMA), a potent inducer of NETosis (T. A. Fuchs et al., 2007, Novel cell death program leads to neutrophil extracellular traps. Journal of Cell Biology 176, 231-241). DNA staining of PMA-stimulated neutrophils showed abundant lattices of extracellular NET-DNA filaments (Fig. 1A). NETs were incubated with ANCA-positive sera. P-ANCA-positive serum prominently stained NETs as evidenced by the co-localization of anti-human-IgG-antibodies with extracellular DNA filaments (Fig. 1B). No staining of NETs was observed with C-ANCA-positive sera (Fig. 1C) or serum without autoantibodies (Fig. 1D). Staining of unstimulated neutrophils was included as a positive control. DNA staining of these cells showed the characteristic lobulated nucleus of neutrophils (Fig. 8). Both C- and P-ANCA-positive sera labeled the cytoplasm, while autoantibody-negative sera did not stain neutrophils (Fig. 8). To exclude the possibility that this finding is restricted to PMA-induced NETs, neutrophils were analyzed, which formed NETs spontaneously. Freshly isolated neutrophils were imaged, which had been incubated for 15 minutes under serum-free conditions without adding a stimulus. As previously shown, under these conditions the formation of NETs occurs in a few cells spontaneously (Fig. 1E and F) ((T. A. Fuchs et al., 2007, Novel cell death program leads to neutrophil extracellular traps. Journal of Cell Biology 176, 231-241). P- and C-ANCA-positive sera showed a cytoplasmic staining pattern in cells with lobulated nuclei (Fig. 1G and H), whereas a NET-staining was observed with P-ANCAs (Fig. 1G). These data indicate that anti-NET-antibodies include P-ANCAs, but not C-ANCAs.

### MPO forms part of NETs, while PR3 resides in netting neutrophils

MPO and PR3 are the predominant antigens of p- and c-ANCAs, respectively (R. J. Falk, J. C. Jennette, 1988, Anti-neutrophil cytoplasmic autoantibodies with specificity for myeloperoxidase in patients with systemic vasculitis and idiopathic necrotizing and crescentic glomerulonephritis, N Engl J Med 318, 1651-1657; J. C. Jennette, J. R. Hoidal, R. J. Falk, 1990, Specificity of anti-neutrophil cytoplasmic autoantibodies for proteinase 3, Blood 75, 2263-2264). Staining of PMA-activated neutrophils with anti-MPO-antibodies showed the co-localization of MPO and NET-DNA fibers (Fig. 2A, 2B). No such staining was observed with anti-PR3-antibodies (Fig. 2C) or unspecific control antibodies (Fig. 2D). Control staining of unstimulated neutrophils showed MPO and PR3 in a dotted cytoplasmic pattern as anticipated due the granular localization of these enzymes (Fig. 9). Using unstimulated neutrophils and spontaneously formed NETs as a substrate (Fig. 2E and F), IFF for MPO and PR3 showed a strong cytoplasmic pattern in cells with lobulated nuclei, whereas NETs stained strongly for MPO (Fig. 2G and H). These data suggest that during NETosis MPO is mobilized from granules to NETs, whereas PR3 resides in the cytoplasm of netting neutrophils.

### Serine protease-inhibition in NETosis generates neutrophils with MPO-positive nuclei

The date obtained confirm a previous report, which showed that Neutrophil Elastase (NE) and MPO, but not PR3, translocate into the nucleus in the initial phases of NETosis (Papayannopoulos V, Metzler KD, Hakkim A, & Zychlinsky A, 2010, Neutrophil elastase and myeloperoxidase regulate the formation of neutrophil extracellular traps. Journal of Cell Biology 191(3):677-691). This study showed that nuclear NE mediates the unfolding of chromatin by cleaving core histones and the linker histone H1. NE activity is therefore required for NET-formation. Neutrophils were stimulated in the presence of DFP, a serine protease inhibitor, which blocks the enzymatic activity of neutrophil serine proteases, including NE (Barna JB & Kew RR, 1995, Inhibition of neutrophil chemotaxis by protease inhibitors. Differential effect of inhibitors of serine and thiol proteases. Inflammation 19(5):561-574; Tsang JL, Parodo JC, & Marshall JC, 2010, Regulation of apoptosis and priming of neutrophil oxidative burst by diisopropyl fluorophosphate. Journal of inflammation 7:32). DFP dose-dependently blocked the generation of NETs and at the highest concentration of DFP all nuclei of PMA-activated neutrophils retained their lobulated shape (Fig. 3A). Interestingly, DFP had no effect on the nuclear translocation of MPO and therefore dose-dependently increased the number MPO-positive nuclei (Fig. 3B). A prolonged incubation time of up to 15 hours was used to achieve a homogenous population of neutrophils with MPO-positive nuclei (data not shown). DFP did not cause a nuclear translocation of PR3 and even at the highest concentration of DFP PR3 remained in the cytoplasm (Fig. 3C). It can be concluded from these data that the inhibition of neutrophil serine proteases with DFP prevents the release of NETs, but does not block the merge of granular enzymes with the nucleus. Accordingly, treatment of PMA-activated neutrophils with DFP enables the generation of a homogenous population of neutrophils, which feature key morphologies of NETosis. Importantly, staining of these "NETotic neutrophils" with P-ANCA-positive sera revealed a nuclear staining (Fig. 4A and B) and C-ANCA-positive sera stained a single cytoplasmic particle (Fig. 4C), whereas autoantibody-negative control sera did not stain NETotic neutrophils (Fig. 4D). These data suggest that during NETosis antigens of P-ANCAs are translocated into the nucleus, while antigens of C-ANCAs reside in the cytoplasm.

### Ethanol-fixation of unstimulated neutrophils mimics NETosis

NETs and NETotic neutrophils were compared to the commercially available substrate for the detection of ANCAs, unstimulated neutrophils fixed with ethanol and formalin. Staining of formalin fixed neutrophils with P-ANCA- and C-ANCA-positive serum gives a cytoplasmic staining pattern, whereas this substrate is not stained by ANAs and autoantibody-negative control serum (Fig. 5A). The fixation with ethanol causes the migration of MPO from the granules into the nucleus, leading to a nuclear staining pattern of P-ANCA-positive serum (Fig. 5B), while C-ANCA-positive serum stains the neutrophil cytoplasm irrespective of whether the cells were fixed with formalin (Fig. 5A) or ethanol (Fig. 5 B). The diagnosis of P-ANCAs is complicated by anti-nuclear-antibodies (ANAs), which avidly stain the nuclei of ethanol-fixed neutrophils, and thus are often indistinguishable from P-ANCAs (Fig. 5B) (Csernok E & Moosig F, 2014, Current and emerging techniques for ANCA detection in vasculitis. Nature Reviews Rheumatology 10(8):494-501). Therefore, ANCAs are detected and differentiated on the commercial substrate by analyzing the staining patterns from ethanol- and formalin-fixed neutrophils. We compared the commercial ANCA-substrates to NETs, generated by PMA-stimulated neutrophils, and NETotic neutrophils, generated by the PMA/DFP-treated neutrophils. Unstimulated neutrophils were included as controls. All cells were fixed with formalin. The unstimulated neutrophils gave a similar staining pattern as the commercial formalin-fixed neutrophils: P- and C-ANCAs stained the cytoplasm, whereas ANA-positive sera and control sera did not stain neutrophils (Fig. 5C). NETs were stained avidly by P-ANCA-positive sera but not by C-ANCAs (Fig. 5D). Given that NETs are rich in nuclear components, such as DNA and histones (Brinkmann V, et al., 2004, Neutrophil extracellular traps kill bacteria. Science 303(5663): 1532-1535), a NET-staining with ANA-positive serum was observed (Fig. 5D). Labeling NETotic neutrophils with P-ANCAs revealed a prominent and homogenous staining of neutrophil nuclei, whereas C-ANCAs stained the PR3 in the cytoplasm (Fig. 5E). Similar to ethanol-fixed unstimulated neutrophils, ANAs stained the nuclei of NETotic neutrophils (Fig. 5E). In conclusion, ethanol-fixation of unstimulated neutrophils produces similar staining patterns as NETotic neutrophils. Therefore ethanol-fixation likely mimics the relocalization of granular enzymes during NETosis.

### Nuclease-treatment prevents the binding of ANA to NETs and nuclei of NETotic neutrophils.

NETs and nuclei ofNETotic neutrophils bind P-ANCAs as well as autoantibodies against nuclear components. Consequently, these substrates cannot discriminate between P-ANCA and ANAs. Neutrophils were stimulated with PMA in the presence or absence of DFP to induce NETs (Fig. 6A) and NETotic neutrophils (Fig. 6C), respectively. The cells were then treated with DNase1, which removed NETs (Fig. 6B) as well as the nuclei of NETotic neutrophils (Fig. 6D). Treatment of NETs with DNase prevented the P-ANCA staining of NETs (Fig. 6B). Interestingly, P-ANCAs and C-ANCAs stained a similar pattern on DNase1-treated NETotic neutrophils (Fig. 6D), suggesting that the antigens of P-ANCAs revert to the cytoplasm if nuclear DNA is degraded. Importantly, DNase1 eliminated the staining of NETs and nuclei of NETotic neutrophils by ANAs (Fig. 6B and D).

### NETs Detect MPO-ANCAs in P-ANCA-Negative Sera.

It was investigated whether NETs may present antigens, which are not exposed on ethanol-fixed neutrophils. To test this, a collection of 15 P-ANCA-negative sera and 15 P-ANCA-positive sera was analyzed. The P-ANCA titers were determined by IIF-screening using the conventional ethanol-fixed neutrophils (Fig. 7A). Importantly, both groups of sera contained similar concentrations of MPO-ANCAs (anti-myeloperoxidase antineutrophil cytoplasmic antibodies) as detected by ELISA with MPO as a substrate (Fig. 7B). At first, the IIF-titer on ethanol-fixed neutrophils was compared with the intensity of the NET-staining. The NET-staining increased with IIF-titers from 80 to 320 and reached a plateau at higher titers (Fig. 7C). Overall, a strong correlation between IIF-titer on the conventional substrate and intensity of the NET-staining was observed (Spearman r = 0.85; p = 0.0001), indicating that both substrates have a comparable sensitivity. Indeed, 14 of 15 P-ANCA-positive sera showed a NET-staining pattern (Fig. 7D), all 15 sera stained nuclei ofNETotic neutrophils (Fig. 7E) and 14 of 15 sera labeled the cytoplasm of DNase1-treated NETotic neutrophils. The one patient serum, which did not label NETs had the lowest P-ANCA-titer on ethanol-fixed neutrophils (1/80). Of 15 ANCA-negative sera, 6 sera stained NETs (Fig. 7D), 10 sera stained the nuclei of NETotic neutrophils (Fig. 7E), and 6 sera stained the cytoplasm of DNase1-treated NETotic neutrophils (Fig. 7F). With four sera a positive staining on all three NET-based substrates was observed. These data suggest that NETs and NETotic neutrophils may present antigens, which are not exposed by ethanol-fixed neutrophils.

## Claims

1. Method for the immunodiagnostic detection of anti-neutrophil antibodies, the method comprising:
a1) Contacting an aliquot of a sample of a body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
a2) Immunolabeling of anti-neutrophil antibodies bound to the stimulated netotic but not netting polymorphonuclear neutrophils.

2. The method according to claim 1, further comprising
b1) Contacting a further aliquot of the sample of body fluid with stimulated netotic but not netting polymorphonuclear neutrophils, PMNs, under conditions allowing binding of anti-neutrophil antibodies in the sample to the polymorphonuclear neutrophils, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, preferably a DNase, further preferred DNase1L3 or DNase I, and
b2) Immunolabeling of anti-neutrophil antibodies bound to the stimulated nuclease-treated netotic but not netting polymorphonuclear neutrophils, and/or
c1) Contacting a further aliquot of the sample of body fluid with Neutrophil Extracellular Traps, NETs, formed by stimulated polymorphonuclear neutrophils, PMNs, under conditions allowing binding of anti-neutrophil antibodies in the sample to the NETs, and
c2) Immunolabeling of anti-neutrophil antibodies bound to the NETs, and/or
d1) Contacting a further aliquot of the sample of body fluid with unstimulated formalin-fixed polymorphonuclear neutrophils, PMNs, under conditions allowing binding of anti-neutrophil antibodies in the sample to the unstimulated formalin-fixed polymorphonuclear neutrophils, and
d2) Immunolabeling of anti-neutrophil antibodies bound to the unstimulated formalin-fixed polymorphonuclear neutrophils.

3. The method according to one of the preceding claims, the method comprising
i) comparing the staining pattern obtained in step a2 with staining patterns obtained for a negative and/or a positive control, or
ii) comparing the staining patterns obtained in steps a2 and b2 with each other and with staining patterns obtained for a negative and/or a positive control, or
iii) comparing the staining patterns obtained in steps a2, b2 and c2 with each other and with staining patterns obtained for a negative and/or a positive control, or
iv) comparing the staining patterns obtained in steps a2 and c2with each other and with staining patterns obtained for a negative and/or a positive control, or
v) comparing the staining patterns obtained in steps a2, b2, c2 and d2 with each other and with staining patterns obtained for a negative and/or a positive control, or
vi) comparing the staining patterns obtained in steps a2, b2 and d2 with each other and with staining patterns obtained for a negative and/or a positive control, or
vii) comparing the staining patterns obtained in steps a2 and d2 with each other and with staining patterns obtained for a negative and/or a positive control.

4. The method of claim 3, wherein the method is at least partially carried out automatically, the method comprising:
i) preparing digital images, preferably binary black and white images, of the staining pattern obtained in step a2 and of the staining patterns of the negative and/or positive controls, and determining and comparing the area and the circularity of the staining patterns obtained in step a2 and of the negative and/or positive controls, or
ii) preparing digital images, preferably binary black and white images, of the staining patterns obtained in steps a2 and b2 and of the staining patterns of the negative and/or positive controls, and determining and comparing the area and the circularity of the staining patterns obtained in steps a2 and b2, and of the negative and/or positive controls, or
iii) preparing digital images, preferably binary black and white images, of the staining patterns obtained in steps a2, b2 and c2, and of the staining pattern of the negative and/or positive control, and determining and comparing the area and the circularity of the staining patterns obtained in steps a2, b2 and c2, and of the negative and/or positive controls, or
iv) preparing digital images, preferably binary black and white images, of the staining patterns obtained in steps a2 and c2, and of the staining patterns of the negative and/or positive controls, and determining and comparing the area and the circularity of the staining patterns obtained in steps a2 and c2, and of the negative and/or positive controls, or
v) preparing digital images, preferably binary black and white images, of the staining patterns obtained in steps a2, b2, c2 and d2, and of the staining patterns of the negative and/or positive controls, and determining and comparing the area and the circularity of the staining patterns obtained in steps a2, b2, c2 and d2, and of the negative and/or positive controls, or
vi) preparing digital images, preferably binary black and white images, of the staining patterns obtained in steps a2, b2 and d2, and of the staining patterns of the negative and/or positive controls, and determining and comparing the area and the circularity of the staining patterns obtained in steps a2, b2 and d2, and of the negative and/or positive controls, or
vi) preparing digital images, preferably binary black and white images, of the staining patterns obtained in steps a2 and d2, and of the staining patterns of the negative and/or positive controls, and determining and comparing the area and the circularity of the staining patterns obtained in steps a2 and d2, and of the negative and/or positive controls.

5. The method according to one of the preceding claims, wherein the stimulated netotic but not netting polymorphonuclear neutrophils and, if applicable, the NETs have been fixed on an object slide, preferably a glass microscope slide, prior to contacting with the body fluid sample.

6. The method according to one of the preceding claims, wherein the sample of body fluid is blood plasma or blood serum.

7. The method according to one of claims 1 to 6, for the differential diagnostic analysis of c-ANCAs, p-ANCAs and preferably ANAs in body fluid samples, preferably blood samples.

8. Method for preparing an object slide for the immunodiagnostic detection of anti-neutrophil antibodies, the method comprising:
a) Generating stimulated netotic but not netting polymorphonuclear neutrophils, PMNs, by treating stimulated polymorphonuclear neutrophils, PMNs, with a NET release inhibiting compound under conditions where the formation of NETs by the stimulated polymorphonuclear neutrophils is suppressed, and
b) Fixing the stimulated netotic but not netting PMNs on an object slide.

9. The method according to claim 8, wherein the fixed stimulated netotic but not netting PMNs are treated with a nuclease, preferably a DNase, further preferred DNase1L3 or DNase I.

10. Object slide or object slide set for the immunodiagnostic detection of anti-neutrophil antibodies, the object slide or object slide set having fixed thereon:
a) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and/or
b) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease.

11. The object slide or object slide set according to claim 10, the object slide or object slide set further having fixed thereon:
c) Neutrophil Extracellular Traps, NETs, formed by stimulated polymorphonuclear neutrophils, PMNs, and/or
d) unstimulated formalin-fixed polymorphonuclear neutrophils, PMNs.

12. The object slide or object slide set according to claim 10 or 11, the object slide or object slide set having fixed thereon:
i) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, or
ii)
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, or
iii)
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, and
- Neutrophil Extracellular Traps, NETs, formed by stimulated polymorphonuclear neutrophils, PMNs, or
iv)
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
- Neutrophil Extracellular Traps, NETs, formed by stimulated polymorphonuclear neutrophils, PMNs, or
v)
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and wherein the stimulated netotic but not netting polymorphonuclear neutrophils have been treated with a nuclease, and
- Neutrophil Extracellular Traps, NETs, formed by stimulated polymorphonuclear neutrophils, PMNs, and
- unstimulated formalin-fixed polymorphonuclear neutrophils, PMNs, or
vi)
- stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound under conditions where the formation of NETs by the stimulated polymorphonuclear neutrophils is suppressed, and
- unstimulated formalin-fixed polymorphonuclear neutrophils, PMNs.

13. Kit for the immunodiagnostic detection of anti-neutrophil antibodies, the kit comprising
a) at least one object slide or object slide set according to claim 10, and
b) at least one fluorescently labeled anti-neutrophil antibody.

14. Use of
a) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound, preferably a serine protease inhibitor, preferably a nucleophil serine protease inhibitor, further preferred a nucleophil elastase inhibitor, and especially preferred diisopropylfluorophosphate, DFP, or phenylmethylsulfonyl fluoride, PMSF, under conditions where the formation of NETs by the stimulated polymorphonuclear neutrophils is suppressed, and/or
b) stimulated netotic but not netting polymorphonuclear neutrophils, PMNs, wherein the netotic but not netting polymorphonuclear neutrophils have been generated by treating stimulated, but not netting, polymorphonuclear neutrophils with a NET release inhibiting compound, preferably a serine protease inhibitor, preferably a nucleophil serine protease inhibitor, further preferred a nucleophil elastase inhibitor, and especially preferred diisopropylfluorophosphate, DFP, or phenylmethylsulfonyl fluoride, PMSF, under conditions where the formation of NETs by the stimulated polymorphonuclear neutrophils is suppressed, and wherein the stimulated netotic but not netting polymorphonuclear neutrophils have been treated with a nuclease, preferably a DNase, further preferred DNase1L3 or DNase I,
for the immunodiagnostic detection of anti-neutrophil antibodies, preferably for the immunodiagnostic detection of anti-neutrophil antibodies in a subject afflicted with or supposed to be afflicted with primary vasculitis, further preferred with granulomatosis with polyangiitis, GPA, or microscopic polyangiitis, MPA.

15. The method for the immunodiagnostic detection of anti-neutrophil antibodies according to one of claims 1 to 7, or the method for preparing an object slide for the immunodiagnostic detection of anti-neutrophil antibodies according to one of claims 8 to 9, or the object slide or object slide set for the immunodiagnostic detection of anti-neutrophil antibodies according to one of claims 10 or 11, wherein
a) the NET release inhibiting compound is a serine protease inhibitor, preferably a nucleophil serine protease inhibitor, further preferred a nucleophil elastase inhibitor, and further preferred diisopropylfluorophosphate, DFP, or phenylmethylsulfonyl fluoride, PMSF, especially preferred DFP and/or
b) stimulated polymorphonuclear neutrophils are obtained by treating naïve polymorphonuclear neutrophils with phorbol 12-myristate 13-acetate, PMA, under conditions where the naive polymorphonuclear neutrophils are stimulated and/or
c) stimulated netotic but not netting polymorphonuclear neutrophils, PMNs, are obtained by stimulating naive PMNs in the presence of the NET release inhibiting compound under conditions that an essentially homogenous population of stimulated netotic but not netting PMNs is obtained, preferably by stimulating the naive polymorphonuclear neutrophils in the presence of DFP under conditions that an essentially homogenous population of stimulated netotic but not netting PMNs is obtained, further preferred by contacting the naive polymorphonuclear neutrophils with 0.1 µM PMA in the presence of 2-20 mM DFP over a period of up to 15 hours at 37°C, and/or
d) the stimulated netotic but not netting polymorphonuclear neutrophils or NETs, if applicable, are derived from PMNs, which have been purified prior to stimulation.
